# EUROPÄISCHE PATENTSCHRIFT

(11) **EP 2 614 352 B1**
(45) Veröffentlichungstag und Bekanntmachung des Hinweises auf die Patenterteilung: **02.05.2018**
(21) Anmeldenummer: 11831823.7
(22) Anmeldetag: 07.09.2011
(51) Int. Cl.: G01N 33/02, G01N 33/04, G01N 30/88, G01N 15/00, G01N 1/22

(54) **VORRICHTUNG ZUR ENTNAHME EINER REPRÄSENTATIVEN UND ZERSTÖRUNGSFREIEN PROBE VON PARTIKELN AUS SCHÜTTGUT SOWIE VERFAHREN ZUR ENTNAHME MITTELS DER VORRICHTUNG**
DEVICE FOR REMOVING A REPRESENTATIVE AND NON-DESTRUCTIVE SAMPLE OF PARTICLES OF BULK MATERIAL AND METHOD FOR REMOVAL USING THE DEVICE
DISPOSITIF DE PRÉLÈVEMENT D'UN ÉCHANTILLON REPRÉSENTATIF ET INTACT DE PARTICULES PROVENANT DE MARCHANDISES EN VRAC, ET PROCÉDÉ POUR EFFECTUER UN PRÉLÈVEMENT À L'AIDE DE CE DISPOSITIF

(30) Priorität: 09.09.2010 DE 102010037425
(43) Veröffentlichungstag der Anmeldung: 17.07.2013
(73) Patentinhaber: Eurofins WEJ Contaminants GmbH, 21079 Hamburg (DE)
(72) Erfinder: BISELLI, Scarlett, 22765 Hamburg (DE); NKENGFACK, Ngnintendem James, 72406 Besingen (DE)
(74) Vertreter: Baudisch, Heidi
(86) Internationale Anmeldenummer: PCT/DE2011/075213
(87) Internationale Veröffentlichungsnummer: WO 2012/048695

(56) Entgegenhaltungen:
- DE-A1- 10 309 354
- US-A- 6 082 179
- US-A- 6 103 534
- STROKA J ET AL: "Novel sampling methods for the analysis of mycotoxins and the combination with spectroscopic methods for the rapid evaluation of deoxynivalenol contamination", TOXICOLOGY LETTERS, ELSEVIER BIOMEDICAL PRESS, AMSTERDAM, NL, Bd. 153, Nr. 1, 10. Oktober 2004 (2004-10-10), Seiten 99-107, XP004549281, ISSN: 0378-4274, DOI: 10.1016/J.TOXLET.2004.04.039
- THOMAS B WHITAKER: "Standardisation of mycotoxin sampling procedures: an urgent necessity", FOOD CONTROL, BUTTERWORTH, LONDON, GB, Bd. 14, 1. Januar 2003 (2003-01-01), Seiten 233-237, XP007920624, ISSN: 0956-7135, DOI: 10.1016/S0956-7135(03)00012-4

## Beschreibung

Die vorliegende Erfindung betrifft ein Verfahren zum Nachweis mindestens einer Mykotoxinkontamination in Schüttgut, weiterhin beschrieben wird eine Vorrichtung zur Entnahme einer Probe von Partikeln aus Schüttgut sowie ein Verfahren zur Entnahme von Partikeln aus Schüttgut mittels der beschriebenen Vorrichtung und die Verwendung der beschriebenen Vorrichtung.

Mykotoxine sind sekundäre Stoffwechselprodukte aus Schimmelpilzen. Bereits in geringsten Mengen wirken Mykotoxine giftig. Insofern stellen Mykotoxine in Lebens- und Futtermitteln ein ernstzunehmendes Gesundheitsrisiko dar. Die Wirkungen von Mykotoxinen auf Säugetiere können sehr unterschiedlich sein. Bekannt ist, dass diese unter anderem eine karzinogene, neurotoxische oder immunsuppressive Wirkung bei Säugetieren entfalten können. Darüber hinaus sind auch allergische Reaktionen auf Mykotoxine beschrieben worden. Bislang sind zahlreiche Mykotoxine bekannt, die von verschiedenen Pilzarten produziert werden können. Bekannte Mykotoxine sind unter anderem Aflatoxine, Mutterkornalkaloide, Ochratoxine A (OTA), Fusarium-Toxine wie Deoxynivalenol (DON) und Zearalenon (ZEA) und Penicillium-Toxine wie Citrinin. Hierbei stellen ZEA und DON zum Beispiel die Hauptmykotoxine in Getreide dar, OTA ist das Hauptmykotoxin in Rohkaffee, während Nüsse überwiegend mit Aflatoxinen belastet sind. Aufgrund der starken Gesundheitsgefahr, die von Mykotoxinen ausgeht, wurden entsprechend in den vergangenen Jahren intensive Bemühungen gemacht, in diesem Bereich Analysenmethoden zu entwickeln und zu verbessern. Generell kommen sowohl flüssigchromatographische Verfahren mit massenspektrometrischer bzw. Fluoreszenzdetektion zum Einsatz als auch Immunoassays (Schuhmacher et al., 2008; Shephard, 2009; Maragos und Busman, 2010). Neben Mykotoxine können Lebensmittel darüber hinaus mit anderen Kontaminationen oder Verunreinigungen belastet sein. Beispiele hierfür sind toxische Metalle oder Metallverbindungen, wie Arsen, Antimon oder Zink, Pflanzenschutzmittel, wie Pestizide, Fungizide, Insektizide oder Herbizide oder gentechnisch veränderte Organismen.

Aufgrund der Tatsache, dass die Mykotoxine sowie andere Kontaminationen oder schädliche Rückstände äußerst heterogen in Lebensmitteln und Futtermitteln verteilt sind, stellt insbesondere die Entnahme und Vorbereitung einer Probe aus den zu untersuchenden Lebensmitteln eine große Herausforderung dar (Biselli et al., Mycotoxin Res. 24 (2), (2008), 98-104; Miraglia et al., Food Addit. Contam. Suppl 1, (2005), 31-36). Die derzeit lebensmittelrechtlich vorgeschriebene Vorgehensweise nach der Verordnung EC 401/2006 "Methods of sampling and analysis for the official control of the levels of mycotoxins in foodstuffs" sieht die Homogenisierung mehrerer Kilogramm des losen Schüttguts, der so genannten Bulkware, vor. Da viele Matrixbestandteile die Analysenverfahren stören und gegebenenfalls insbesondere eine Quantifizierung der Mykotoxine erschweren oder sogar verhindern, schließen sich an die Homogenisierung üblicherweise recht teure und aufwändige Aufreinigungsverfahren an, wie Festphasenextraktion, Festphasenmikroextraktion oder Affinitätschromatograhie (Senyuva und Gilbert, J Chromatogr. B Analyt Technol Biomed Life Sci, 878 (2), (2010), 115-32; Biselli und Hummert, Food Addit. Contam. 22 (8), (2005), 752-60). Probenahme und Probenvorbereitung zeichnen sich daher insgesamt durch hohe Zeit- und Arbeitsintensität aus.

Für eine einfachere Probennahme sind bereits verschiedene Probenahmelanzen beschrieben. In der EP 0 411 932 B1 ist eine Probensonde beschrieben zur Entnahme einer repräsentativen Teilprobe aus Getreide und Mehl. In der EP 1 221 605 B1 ist eine tragbare speerförmige Probenahmelanze beschrieben zur Entnahme einer Staubprobe.

Die US 6,324,927 B1 beschreibt eine Methode und Vorrichtung für die Probenahme von Kontaminanten, wonach die Probe in einer luftdichten Kammer in Anwesenheit eines Trägergases Vibrationen ausgesetzt wird, die zur Freisetzung von Staubpartikeln führen.

Stroka et al., Toxicology Letters, 153 (2004), 99-107 beschreiben die Probenahme verschiedener Lebensmittelbulkwaren mit Hilfe einer Probenahmelanze bzw. dem DiscoveryCERT FQS™ Probenahmesystems. Der Staubanteil der Probe wird auf verschiedenen Filtermaterialien gesammelt und mittels High Performance Liquid Chromatography (HPLC)- sowie Fourier-Transformations-IR-Spektroskopie (FTIR)-Methoden analysiert. Eine klare und eindeutige Korrelation der Kontaminationen von Staub und Gesamtprobe kann jedoch nicht festgestellt werden.

Nachteilig an den bislang bekannten Methoden ist, dass diese Verfahren eine Probengewinnung über Glasfaserfilter bzw. über Membranen vorsehen. Diese Art der Probensammlung ermöglicht jedoch nur eine Auswertung dieser Proben in Form einer Einzelanalyse. Eine erneute Überprüfung oder Wiederholung der Analyse der gesammelten Probe ist somit nicht möglich. Weiterhin kann durch die so gewonnenen Proben keine eindeutige Korrelation im Hinblick auf die Belastung durch Mykotoxine oder andere Kontaminationen bei der entnommenen Probe im Vergleich zu der Gesamtprobe nachgewiesen werden. Insofern stellen diese mit den bislang verfügbaren Methoden gewonnenen Proben keine repräsentativen Proben dar. Dadurch ist die Aussagekraft einer Probe, die keinen eindeutigen Anhaltspunkt über die tatsächliche Kontamination mit Mykotoxinen oder anderen Kontaminationen bietet, deutlich geschmälert. Weiterhin nachteilig an den bisher bekannten Verfahren ist zudem, dass diese teilweise technisch sehr aufwendig sind. Dies bringt potentielle Fehlerquellen mit sich und verursacht außerdem hohe Kosten in der Anschaffung und dem Betrieb solcher Vorrichtungen.

Im Hinblick auf diese vorgenannten Nachteile der Methoden des bekannten Stands der Technik wird eine verbesserte und repräsentative Probenentnahme von Lebensmittel- und Futtermittelschüttgut, so genannter Bulkware beschrieben. Weiterhin wird ein einfach durchzuführendes Verfahren beschrieben, das kostengünstig und weniger arbeitsintensiv ist. Der vorliegenden Erfindung liegt das technische Problem zugrunde, ein verlässliches und aussagekräftiges Verfahren zum Nachweis einer Mykotoxinkontamination bereitzustellen.

Dieses zugrunde liegende technische Problem wird durch den in den Ansprüchen definierten Gegenstand gelöst.

Ein erster Gegenstand der vorliegenden Erfindung betrifft ein Verfahren zum Nachweis mindestens einer Mykotoxinkontamination in Schüttgut umfassend die Schritte: (a) Entnehmen einer Probe von Partikeln mit einer Partikelgröße von 0,1 mm und 1,0 mm aus Schüttgut; (b) Analysieren der entnommenen Partikel; und (c) Nachweis einer Mykotoxinkontamination durch Bestimmen der Konzentration der Mykotoxinkontamination, und (d) Rückschluss auf die tatsächliche Mykotoxinkontamination des Schüttguts durch Korrelieren der in Schritt (c) bestimmten Konzentration in der Probe mit derjenigen einer Gesamtprobe des Schüttguts.

Erfindungsgemäß werden unter "Kontaminationen" insbesondere Verunreinigungen von Lebensmitteln verstanden. Solche Verunreinigungen umfassen Mykotoxine, toxische Metalle oder Metallverbindungen, wie Arsen, Antimon oder Zink, Pflanzenschutzmittel, wie Pestizide, Fungizide, Insektizide oder Herbizide oder gentechnisch veränderte Organismen.

Mykotoxine umfassen vor allem Ochratoxine, Aflatoxine, Mutterkornalkaloide, Fusarium-Toxine, wie insbesondere Deoxynivalenol (DON) und Zearalenon (ZEA), Alternaria-Toxine und Citrinin.

Erfindungsgemäß bevorzugt sind die Mykotoxine in dem erfindungsgemäßen Verfahren zum Nachweis mindestens einer Mykotoxinkonzentration ausgewählt aus der Gruppe bestehend aus Ochratoxinen, Aflatoxinen, Mutterkornalkaloiden, Fusarium-Toxinen, wie insbesondere Deoxynivalenol und Zearalenon, Alternaria-Toxinen und Citrinin.

In einer bevorzugten Ausführungsform der Erfindung ist vorgesehen, dass die Partikelgröße der entnommenen Probe eine Größe von 0,1 mm bis 0,7 mm, weiter bevorzugt von 0,2 mm bis 0,5 mm aufweist.

In einer weiteren bevorzugte Ausführungsform wird die Analyse mittels High Performance Liquid Chromatography (HPLC), Ultra-High-Performance Chromatography (UPLC), Fourier-Transformations-IR-Spektroskopie (FTIR), Kernspinresonanzspektroskopie (nuclear magnetic resonance, NMR), Direct Analysis in Real Time (DART) oder Enzyme-linked immunosorbent assay (ELISA) durchgeführt.

Weiterhin beschrieben ist eine Vorrichtung zur Entnahme einer Probe von Partikeln aus Schüttgut mit einer Trenneinheit (100) und einer Saugeinheit (110), um die Probe durch einen von der Trenneinheit hin zu der Saugeinheit führenden Luftstrom zu entnehmen, umfassend einen Kanal (20) mit einer stromaufwärts ausgerichteten Öffnung (25), in der eine Sonde (30) angeordnet ist mit einem Sieb (35), einem von dem Kanal (20) stromabwärts und senkrecht zu dem Kanal (20) angeordneten Fliehkraftabscheider (40) mit einem unterhalb des Fliehkraftabscheider (40) angebrachten Behälter (45), einem in den Fliehkraftabscheider (40) hineinführenden Kanal (50), der stromabwärts zu einem Filter (55) führt, einem stromabwärts von dem Filter (55) abführenden Kanal (60), der von dem Filter (55) bis zur Saugeinheit (110) führt, mit einem Ventil (65) und einem von dem Kanal (60) abzweigenden Kanal (70) mit einem am Ende des Kanals (70) angeordneten Manometer (75), wobei die Saugeinheit (110) ein Luftgebläse ist, das in dem Kanal (20) eine Geschwindigkeit des Luftstroms von bevorzugt 30 bis 70 m/s erzeugt.

Im Zusammenhang mit der vorliegenden Erfindung wird unter "Schüttgut" ein Gemenge von körniger oder stückiger loser einzelner Ware verstanden, das in schüttfähiger Form vorliegt. Erfindungsgemäß wird unter "Schüttgut" insbesondere Lebens- und Futtermittelschüttware, wie Körner von Getreide, beispielsweise Weizen, Mais, Roggen oder Gerste, sowie Mehl, Salz, Zucker, Nüsse oder Kaffee verstanden.

Hierin beschrieben ist, dass unter "stromaufwärts" eine Orientierung verstanden wird, die im räumlichen Verhältnis zu der Saugeinheit und dem darin erzeugten Luftstrom hinsichtlich der Länge einen größeren Abstand aufweist. Entsprechend wird unter "stromabwärts" eine Orientierung verstanden, die im räumlichen Verhältnis zu der Saugeinheit und dem darin erzeugten Luftstrom hinsichtlich der Länge einen geringeren Abstand aufweist.

Unter "senkrecht" wird eine Ausrichtung verstanden, die im Wesentlichen einen Winkel von 90° darstellt. Weiterhin wird unter "senkrecht" auch eine Ausrichtung verstanden, die einen von einem 90° Winkel abweichenden Winkel, beispielsweise in einem Bereich von 45° bis 135°, vorsieht. Es ist vorgesehen, dass unter einer senkrechten Ausrichtung eine solche Ausrichtung verstanden wird, in der der Kanal (20) im Verhältnis zu dem Fliehkraftabscheider (40) so ausgerichtet ist, dass der Luftstrom mit den Partikeln so auf den Fliehkraftabscheider (40) trifft, dass die Partikel tangential eingebracht werden und dadurch auf eine kreisförmige Bahn in dem Fliehkraftabscheider gelangen.

Unter einem "Kanal" wird eine Verbindung, insbesondere eine röhrenartige Verbindung wie ein Rohr, ein Schlauch oder eine Leitung, verstanden.

Kontaminationen, wie solche durch Mykotoxine, sind gewöhnlich sehr inhomogen im Schüttgut verteilt. Das bedeutet, dass Kontaminationen, insbesondere Mykotoxine, uneinheitlich und unregelmäßig verteilt in dem Schüttgut vorliegen. Somit kann nicht davon ausgegangen werden, dass bereits durch die Entnahme einer beliebigen Probe eine Kontamination korrekt nachgewiesen werden kann, da eine hohe Wahrscheinlichkeit besteht, dass diese Probe tatsächlich nur wenig belastet ist. Dies kann jedoch unter Umständen nicht die tatsächliche Situation wiedergeben, da das Gesamtschüttgut möglicherweise deutlich stärker kontaminiert ist als dies in einer einzelnen Probe bestimmt worden ist. So muss durch die inhomogene Verteilung der Kontaminationen befürchtet werden, dass Einzelproben jeweils unterschiedliche Konzentrationen der Kontaminationen wiedergeben. Damit ist jedoch davon auszugehen, dass eine Einzelprobe an sich nicht aussagekräftig und repräsentativ ist, um ausgehend davon auf die tatsächliche Kontamination des kompletten Schüttguts zu schließen.

Insofern bestehen Vorbehalte gegenüber einem Verfahren, das auf der Entnahme einer einfachen, kleineren Probe, wie einer Partikelprobe beruht, um dadurch die Kontamination des kompletten Schüttguts zu bestimmen.

Überraschenderweise ist es jedoch durch das erfindungsgemäße Verfahren zum Nachweis mindestens einer Kontamination in Schüttgut, möglich, bereits durch die Entnahme einer Partikelprobe mit einer Größe von 0,1 mm bis 1,0 mm einen verlässlichen und aussagekräftigen Nachweis einer Kontamination zu ermöglichen. Es konnte gezeigt werden, dass die Partikelprobe eine Kontamination aufweist, die sich in ihrer Konzentration zwar von der Kontamination der Gesamtprobe unterscheidet. Insofern kann die Konzentration der Kontamination der Partikelprobe beispielsweise deutlich höher sein als dies in der Gesamtprobe der Fall ist. Erstaunlicherweise konnte gezeigt werden, dass die entnommene Partikelprobe mit einer Partikelgröße von 0,1 mm bis 1,0 mm eine repräsentative Partikelprobe darstellt, die Aufschluss über die tatsächliche Konzentration der Kontamination der Gesamtprobe gibt. Durch eine Korrelation der Konzentration der Kontamination der Partikelprobe mit derjenigen der Gesamtprobe konnte bestätigt werden, dass die Partikelprobe einen Rückschluss auf die tatsächliche Kontamination erlaubt.

Erfindungsgemäß wird unter einem "verlässlichen Nachweis" bzw. unter einem "aussagekräftigen Nachweis" ein Nachweis verstanden, der das Vorliegen einer Lebensmittelkontamination in Schüttgut in einem signifikanten Maß bestimmt, so dass ein nach statistischen Grundlagen gesichertes Ergebnis erreicht wird.

Überraschenderweise konnte weiter gezeigt werden, dass durch die hierin beschriebene Vorrichtung Proben einer bestimmten Partikelgröße gewonnen werden können, die nach der Analyse auf Mykotoxine als Kontamination in einer bestimmten Mykotoxinkonzentration in einem hohen Maße mit der Mykotoxinkonzentration der Gesamtprobe des untersuchten Schüttguts korrelieren. Diese vorteilhafte repräsentative Probenentnahme wird durch die Gewinnung der Probe durch den Fliehkraftabscheider, auch Zyklon genannt, ermöglicht. Im Zusammenhang mit der vorliegenden Erfindung wird unter "repräsentativ" die Eigenschaft einer Probe verstanden, bestimmte Charakteristika der Probe, wie Kontaminationen mit Mykotoxinen oder pflanzlichen oder mikrobiellen Toxinen, so widerzuspiegeln, dass diese Probe mit der Gesamtprobe hinsichtlich bestimmter Charakteristika in einem hohen Maße korreliert. Der Fliehkraftabscheider ermöglicht eine Absonderung von Partikeln, die in dem von der Saugeinheit erzeugten Luftstrom enthalten sind. Es ist beschrieben, dass der Fliehkraftabscheider eine kegelförmige Gestalt aufweist, die sich nach unten verjüngt. Weiterhin wird beschrieben, dass in den Fliehkraftabscheider tangential das Luftstrom/Partikelgemisch eingebracht wird. Dadurch werden die in dem Luftstrom enthaltenen Partikel in eine kreisförmige Bahn gelenkt. Durch die Verjüngung der kegelförmigen Gestalt des Fliehkraftabscheiders nimmt die Drehgeschwindigkeit des Luftstroms zu, so dass die Partikel durch die entstehende Fliehkraft an die Kegelwand des Fliehkraftabscheiders geschleudert werden. Somit wird beschrieben, dass solche Partikelproben aus dem zu untersuchenden Schüttgut entnommen werden, die eine bestimmte Partikelgröße besitzen.

Es wird weiterhin beschrieben, dass mit dem Fliehkraftabscheider das Einstellen eines Luftstroms mit einer bestimmten Saugkraft ermöglicht wird, wodurch gezielt Partikel mit einer gewünschten Größe isoliert und entnommen werden können. Es konnte gezeigt werden, dass durch die hierin beschriebene Vorrichtung Partikel mit einer bestimmten Partikelgröße isoliert werden können, die eine hohe Mykotoxinbelastung aufweisen. Mykotoxine siedeln sich im besonderen Maße an der Oberfläche an. Partikel mit einem geringeren Durchmesser besitzen ein größeres Oberfläche/Volumen-Verhältnis im Vergleich zu Partikeln mit einem größeren Durchmesser. Somit sind Partikel mit einer geringeren Größe mit einem größeren Volumen/Oberfläche-Verhältnis in einem höheren Maße mit Mykotoxinen belastet als Partikel mit einer größeren Größe und einem vergleichsweise kleineren Volumen/Oberfläche-Verhältnis. Durch die hierin beschriebene Vorrichtung können daher insbesondere Proben in einem Partikelgrößenbereich gewonnen werden, die im hohen Maße in ihrer Mykotoxinkonzentration die Gesamtbelastung mit Mykotoxinen in der Schüttware repräsentieren. Somit ist wird durch die hierin beschriebene Vorrichtung in vorteilhafter Weise erreicht, dass durch die Gewinnung von Proben einer bestimmten Partikelgröße sehr repräsentative Proben isoliert werden können, wodurch auf einfache und verlässliche Weise die Belastung mit Mykotoxinen in der gesamten Schüttware festgestellt werden kann.

Es wird eine Vorrichtung beschrieben, in der die Trenneinheit (100) im Wesentlichen einen Fliehkraftabscheider (40), einen Behälter (45), einen Kanal (50), einen Filter (55), ein Ventil (65), einen Kanal (70) und einen Manometer (75) umfasst.

Es wird weiterhin beschrieben, dass die Sonde (30) in eine Mobilisierungseinheit (130) mit einer Öffnung (135) eingeführt wird, aus der die Probe des Schüttguts gewonnen wird.

Es wird hierin beschrieben, dass die Mobilisierungseinheit (130) ein Trommelmischer ist, der vorzugsweise eine Drehzahl von 10 bis 120 rpm, bevorzugt von 15 bis 100 rpm, weiter bevorzugt von 15 bis 80 rpm, weiter bevorzugt von 15 bis 50 rpm, weiter bevorzugt von 15 bis 30 rpm, insbesondere bevorzugt von 28 rpm aufweist. Es wird beschrieben, dass solche Drehzahlen eine zerstörungsfreie Entnahme der Probe aus der Schüttware ermöglichen. Es wird unter "zerstörungsfrei" die Eigenschaft einer Probe verstanden, in einem intakten und kompletten Zustand vorzuliegen.

Es wird beschrieben, dass die Mobilisierungseinheit (130) ein Rüttler, ein Rüttelband oder ein von einem Gebläse erzeugter Luftstrom ist.

Die Mobilisierungseinheit (130) besteht aus Eisen oder Stahl mit einem Motor. Weiterhin beschrieben werden Metalle, Metalllegierungen oder auch Kunststoffe, Polymere.

Die Mobilisierungseinheit (130) weist einen Durchmesser von 20 bis 100 cm, weiter von 30 bis 60 cm, insbesondere von 50 cm auf.

Es wird beschrieben, dass die Sonde (30) einen Durchmesser von 2 cm bis 25 cm, weiter von 4 cm bis 10 cm, insbesondere von 7 cm aufweist. Es wird beschrieben, dass durch eine Vergrößerung des Sondendurchmessers eine Vergrößerung des Partikelabstandes erreicht werden kann. Somit ermöglicht die Verwendung einer Sonde mit größerem Durchmesser, dass dadurch auch ein weiterer Abstand zu den aufzunehmenden Partikeln eingehalten werden kann. Dadurch kann mit einem räumlich flexiblen Abstand eine Partikelprobe entnommen werden, ohne dass die Notwendigkeit besteht, die Sonde in unmittelbarer Nähe zu dem zu untersuchenden Schüttgut platzieren zu müssen. Die Sonde (30) besteht aus Polyethylen (PE).

Alternativ werden weitere Materialien aus Kunststoff, Polymeren, Glas oder Metall beschrieben.

Es wird beschrieben, dass das Sieb (35) in der Sonde (30) ein Metallsieb ist. Es wird weiter beschrieben, dass das Sieb (35) aus Kunststoff oder einer Membran besteht. Beschrieben wird, dass das Sieb (35) eine Maschenweite von 1 mm bis 10 mm, weiter von 2 mm bis 5 mm, insbesondere von 3 mm aufweist.

In einer bevorzugten Ausführungsform der Erfindung ist vorgesehen, dass die Partikelgrößenfraktion der gesammelten Probe bevorzugt eine Größe von 0,1 mm bis 1,0 mm, weiter bevorzugt 0,1 mm bis 0,7 mm, insbesondere bevorzugt 0,2 mm bis 0,5 mm aufweist. Erfindungsgemäß bevorzugt sind somit Partikel der gesammelten Probe mit einer Größe von 0,1; 0,2; 0,3; 0,4; 0,5; 0,6; 0,7; 0,8; 0,9 oder 1,0 mm.

Es wird beschrieben, dass die Trenneinheit (100) ein Außenmaß von 10 cm bis 100 cm, weiter bevorzugt von 20 cm bis 60 cm, insbesondere bevorzugt von 40 cm aufweist.

Es wird weiter beschrieben, dass die Saugeinheit (110) ein Luftgebläse ist, das in dem Kanal (20) eine Geschwindigkeit des Luftstroms von bevorzugt 40 bis 60 m/s, insbesondere von 58 m/s erzeugt. Weiterhin wird beschrieben, dass die Saugeinheit (110) ein Außenmaß von 10 cm bis 100 cm, weiter von 20 cm bis 60 cm, insbesondere von 40 cm aufweist.

Es wird beschrieben, dass durch das Einstellen eines Luftstroms mit einer bestimmten Saugkraft die Abscheidung einer bestimmten Partikelfraktion im Fliehkraftabscheider erreicht wird. Diese Einstellung wird durch das Reduzierventil erreicht. Die Kontrolle der Einstellung des Saugstroms erfolgt durch den Manometer. Durch den ermittelten Druck kann entsprechend ein Luftstrom mit einer bestimmten Saugkraft eingestellt werden.

Es wird beschrieben, dass der Kanal (20) aus Kunststoff besteht. Andere Materialien sind Polymere, Glas, Metalle oder Metalllegierungen. Weiterhin beschrieben wird, dass der Kanal (20) einen Durchmesser von 1 cm bis 5 cm, weiter bevorzugt von 1,5 cm bis 3,5 cm, insbesondere bevorzugt von 2,5 cm aufweist. Weiterhin wird beschreiben, dass der Kanal (20) eine Länge von 0,1 m bis 5 m, weiter von 0,1 m bis 3 m, insbesondere von 0,1 m bis 2 m aufweist. Es wird beschrieben, dass der Kanal (20) an seinem stromabwärts ausgerichteten Ende so ausgestaltet ist, dass ein tangentiales Einblasen des Partikel-/Luftstromgemisches auf eine kreisförmige Bahn in den Fliehkraftabscheider (40) ermöglicht wird.

Es wird beschrieben, dass der Fliehkraftabscheider (40) aus Polyethylen besteht. Weiterhin wird beschrieben, dass der Fliehkraftabscheider (40) aus anderen Kunststoffen oder Polymeren, Glas, Metallen oder Metalllegierungen besteht. Der beschriebene Fliehkraftabscheider (40) weist einen Durchmesser von 1 cm bis 25 cm, weiter von 5 cm bis 10 cm, insbesondere von 8 cm auf. In einem weiteren Beispiel weist der Fliehkraftabscheider (40) eine Höhe von 1 cm bis 50 cm, weiter von 1 cm bis 30 cm, insbesondere von 20 cm auf.

Es wird beschrieben, dass der Behälter (45) ein Gefäß ist, in dem die Proben gesammelt werden, also ein Probensammelgefäß ist. In einem Beispiel wird beschrieben, dass der Behälter (45) über ein Schraubgewinde mit dem Fliehkraftabscheider (40) verbunden ist. Andere Verbindungen, wie über einen Schliff an dem Fliehkraftabscheider oder an dem Behälter, ebenso wie eine Klammer werden weiterhin beschrieben. Es wird beschrieben, dass der Behälter (45) aus Polyethylen (PE) besteht. Weiterhin wird beschrieben, dass der Behälter (45) aus Kunststoff, Polymeren oder Glas besteht. Es wird weiterhin beschrieben, dass der Behälter (45) ein Volumen von 5 ml bis 500 ml, weiter von 100 ml bis 400 ml, insbesondere von 200 ml aufweist.

In einem weiteren Beispiel wird beschrieben, dass in dem Behälter (45) ein Extraktionsmittel vorgelegt ist. Mögliche Extraktionsmittel sind Acetonitril, Methanol oder Wasser.

Weiterhin werden Mischungen von zwei oder drei verschiedenen Extraktionsmitteln beschrieben. In einem weiteren Beispiel wird beschrieben, dass die zwei oder drei verschiedenen Extraktionsmittel in verschiedenen Anteilen zueinander in der Mischung vorliegen können.

In einem Beispiel wird ein Acetonitril/Wasser Gemisch als Extraktionsmittel verwendet. In einem Beispielist das Mischungsverhältnis von Acetonitril/Wasser 80/20 v/v, weiter Acetonitril/Wasser 60/40 v/v, weiter Acetonitril/Wasser 50/50 v/v, weiter Acetonitril/Wasser 40/60 v/v, weiter Acetonitril/Wasser 20/80 v/v, und weiter reines Wasser.

In einem weiteren Beispiel wird beschrieben, dass das Extraktionsmittel Acetonitril/Methanol/Wasser ist. Weiterhin wird beschrieben, dass Acetonitril/Methanol/Wasser mit einem Mischungsverhältnis von 40/40/20 v/v/v vorliegt.

Es wird beschrieben, dass das Extraktionsmittel entsprechend dem nachzuweisenden Toxin bzw. dem zu untersuchenden Material angepasst werden kann.

In einem weiteren Beispiel wird beschrieben, dass unmittelbar an den Behälter (45) eine Analysevorrichtung angeschlossen ist.

Weiterhin bevorzugt ist erfindungsgemäß vorgesehen, dass die Analysevorrichtung zur Durchführung von chromatographischen, spektroskopischen oder immunologischen Verfahren geeignet ist.

Erfindungsgemäß bevorzugt ist vorgesehen, dass die Analysevorrichtung zur Durchführung von High Performance Liquid Chromatography (HPLC), Ultra-High-Performance Liquid Chromatographie (UPLC), Fourier-Transformations-IR-Spektroskopie (FTIR), Kernspinresonanzspektroskopie (nuclear magnetic resonance, NMR), Direct Analysis in Real Time (DART) oder Immunoassay, insbesondere Enzyme-linked immunosorbent assay (ELISA) geeignet ist.

In einem weiteren Beispiel wird beschrieben, dass der Kanal (50) an seinem stromaufwärts gelegenen Ende senkrecht in den Fliehkraftabscheider (40) in Form eines Tauchrohrs hineinführt. Es wird beschrieben, dass der Kanal (50) aus Kunststoff besteht. Weiterhin wird beschrieben, dass der Kanal (50) einen Durchmesser von 1 cm bis 5 cm, weiter von 2 cm bis 4 cm, insbesondere von 2,5 cm aufweist.

In einem weiteren Beispiel wird beschrieben, dass der Filter (55) ein Glasfaserfilter ist. Es wird beschrieben, dass der Filter (55) auf einer Metallfritte in einem Filterhalter liegt. Es wird beschrieben, dass der Filter (55) eine Verschmutzung der Saugeinheit (110) verhindert. In einem Beispiel wird beschrieben, dass der Filterhalter einen Durchmesser von 1 cm bis 20 cm, weiter von 1 cm bis 15 cm, insbesondere von 8 cm aufweist. Weiterhin wird beschrieben, dass der Filter (55) eine Höhe von 1 cm bis 20 cm, weiter von 1 cm bis 15 cm, insbesondere von 8 cm aufweist.

In einem Beispiel wird beschrieben, dass der Kanal (70) aus Kunststoff ist. Weiterhin wird beschrieben, dass der Kanal (70) einen Durchmesser von 1 cm bis 5 cm, weiter von 2 cm bis 4 cm, insbesondere von 2,5 cm aufweist.

In einem Beispiel wird beschrieben, dass der Kanal (60) ein Schlauch ist. In einem Beispiel ist der Kanal (60) ein Gummischlauch.

In einem weiteren Beispiel wird beschrieben, dass das Ventil (65) ein Reduzierventil ist. Es wird beschrieben, dass über das Ventil (65) der von der Saugeinheit (110) ausgehende Luftstrom reguliert wird.

Es wird beschrieben, dass der Fliehkraftabscheider (40), der Behälter (45), der Kanal (50), der Filter (55), der Kanal (70), der Manometer (75), das stromabwärts gerichtete Ende des Kanals (20) sowie das stromaufwärts gerichtete Ende des Kanals (60) in einer Kammer (10) angeordnet sind, die eine stromaufwärts gerichtete Öffnung (15) und eine stromabwärts gerichtete Öffnung (16) aufweist, wobei das stromabwärts gerichtete Ende des Kanals (20) durch die Öffnung (15) und das stromaufwärts gerichtete Ende des Kanals (60) durch die Öffnung (16) führt. Es wird beschrieben, dass die Kammer (10) eine Transportfähigkeit und eine Standfestigkeit der Vorrichtung ermöglicht.

Weiterhin wird ein Verfahren zur Entnahme einer Probe von Partikeln aus Schüttgut beschrieben mittels der oben dargestellten Vorrichtung zum Nachweis einer Mykotoxinkontamination, umfassend die Schritte:
(a) In Kontakt Bringen der Sonde (30) mit Partikeln des Schüttguts,
(b) Ansaugen der Partikel durch Erzeugen eines Luftstroms ausgehend von der Saugeinheit (110) stromaufwärts über den Fliehkraftabscheider (40) zu der Sonde (30), und
(c) Auffangen und Entnehmen der Partikel in dem unterhalb des Fliehkraftabscheiders (40) angebrachten Behälter (45), wobei die Partikelgrößenfraktion der gesammelten Probe bevorzugt eine Größe von 0,1 mm bis 1,0 mm aufweist.

Es wird beschrieben, dass die Probe eine Größe von 0,1 mm bis 0,7 mm, und weiter bevorzugt von 0,2 mm bis 0,5 mm aufweist.

Es wird beschrieben, dass die Partikel in Schritt (a) durch Bewegen des Schüttguts verwirbelt werden.

Es wird beschrieben, dass durch die beschriebene Vorrichtung sowie durch das Verfahren kein direkter Kontakt der Sonde durch ein Einführen in das Schüttgut stattfindet. Vielmehr ist vorgesehen, dass ein Kontakt Bringen nur mit Partikeln aus dem Schüttgut erfolgt. Insofern besteht durch die Vorrichtung sowie durch das Verfahren die Möglichkeit, bereits bei einer Be- oder Entladung der Schüttware Partikel zu entnehmen. Es wird beschrieben, dass ein In Kontakt Bringen der Sonde mit den Partikel durch eine Verwirbelung über eine Mobilisierungseinheit, wie einen Trommelmischer, einen Rüttler, ein Rüttelband oder einen durch ein Gebläse erzeugten Luftstrom erreicht wird.

Die Verwendung der beschriebenen Vorrichtung zur Entnahme einer repräsentativen Probe aus Schüttgut zum Nachweis mindestens einer Mykotoxinkontamination, wobei die Partikelgrößenfraktion der gesammelten Probe bevorzugt eine Größe von 0,1 mm bis 1,0 mm aufweist, wird beschrieben.

Weiter bevorzugt ist die Partikelgrößenfraktion der gesammelten Probe eine Größe von 0,1 mm bis 0,7 mm, besonders bevorzugt von 0,2 mm bis 0,5 mm.

Weiterhin wird beschrieben, dass die Verwendung zum Nachweis von Mykotoxinen verwendet wird.

Bevorzugt sind die Mykotoxine ausgewählt aus der Gruppe bestehend aus Ochratoxinen, Aflatoxinen, Mutterkornalkaloiden, Fusarium-Toxinen wie insbesondere bevorzugt Deoxynivalenol und Zearalenon, Alternaria-Toxinen und Citrinin.

Die Erfindung wird anhand der folgenden Beispiele und der dazugehörigen Figuren näher erläutert.
Figur 1 zeigt eine schematische Darstellung eines Beispiels der Vorrichtung.
Figur 2 zeigt die Korrelation der Deoxynivalenol (DON) Konzentration [µg/kg] von Partikelproben, die mit der Vorrichtung gewonnen wurden, und Kontrollproben in Weizen.
Figur 3 zeigt die Korrelation der Zearalenon (ZON) Konzentration [µg/kg] von Partikelproben, die mit der Vorrichtung gewonnen wurden, und Kontrollproben in Weizen.
Figur 4 zeigt die Korrelation der Deoxynivalenol (DON) Konzentration [µg/kg] von Partikelproben, die mit der Vorrichtung gewonnen wurden, und Kontrollproben in Mais.
Figur 5 zeigt die Korrelation der Zearalenon (ZON) Konzentration [µg/kg] von Partikelproben, die mit der Vorrichtung gewonnen wurden, und Kontrollproben in Mais.
Figur 6 zeigt die Korrelation der Ochratoxin A (OTA) Konzentration [µg/kg] von Partikelproben, die mit der Vorrichtung gewonnen wurden, und Kontrollproben in Kaffee.

### Beispiel 1: Entnahme einer repräsentativen und zerstörungsfreien Probe von Partikeln aus Schüttgut mittels einer Vorrichtung

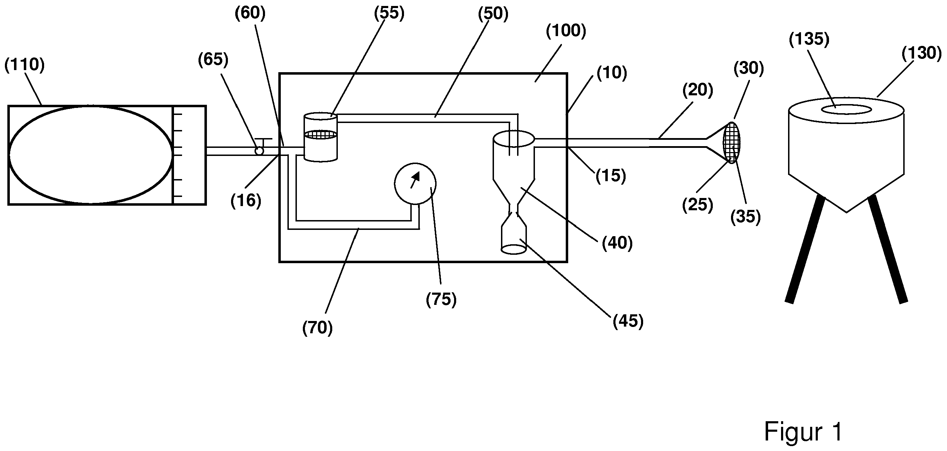

Circa 5 kg lose Schüttware (Lebensmittelbulkware) von Getreide, Rohkaffee oder Nüssen werden in einen Trommelmischer gegeben. Der Trommelmischer wird mit einer Umdrehungszahl von 28 rpm betrieben. Dadurch werden Partikel aus der Schüttware gelöst und in der Umgebungsluft verwirbelt. Die Sonde (30) der Vorrichtung, die in Figur 1 dargestellt ist, wird in die Mobilisierungseinheit (130) in die Öffnung (135) eingeführt. Ausgehend von dem Luftgebläse (110) wird ein Luftstrom generiert, der in dem von der Sonde (30) stromabwärts verlaufenden Rohr (20) eine Geschwindigkeit von 58,2 m/s erzeugt. Dadurch werden die Partikel angesaugt und von der Sonde (30) über das Rohr (20) über den Fliehkraftabscheider (40) bis zum unterhalb des Fliehkraftabscheiders (40) angeordneten Probensammelgefäß (45) geführt. Durch die konkrete Ausgestaltung der Vorrichtung, insbesondere den Fliehkraftabscheider (40), ist vorgesehen, dass insbesondere Partikel mit einer Größe von 0,2 mm bis 0,5 mm gesammelt werden.

### Beispiel 2: Analysenmethode für Mykotoxine aus Lebensmittelschüttgut

### 2.1 Gewinnung der erfindungsgemäßen Partikelprobe

Aus circa 5 kg Lebensmittelbulkware (Weizen, Malz, Mais, Rohkaffee, Nüsse oder ähnliche Schüttwaren) werden Partikel gemäß dem in Beispiel 1 beschriebenen Verfahren mit der Vorrichtung gewonnen.

### 2.2 Probenvorbereitung der gesammelten erfindungsgemäßen Partikelprobe zur Bestimmung der Mykotoxinkonzentration

0,25 - 0,5 g der Partikelprobe werden mit 10 ml eines Acetonitril/Wasser-Gemisches (80:20; v:v) versetzt. Als interne Standards werden 200 µl einer Zearalenon-Lösung mit einer Konzentration von c = 5 µg/ml sowie 200 µl einer FusarenonX-Lösung mit einer Konzentration von c = 20 µg/ml dotiert. Die Extraktion erfolgt 5 Minuten im Ultraschallbad bei höchster Leistungsstufe sowie 20 Minuten auf der Schüttelmaschine.

Der Extrakt wird über einen Glasfaserfilter filtriert. Ein Aliquot des Extraktes wird bei 60°C am Stickstoffstrom eingeengt und im HPLC-Eluenten wieder aufgenommen und 1:5 mit HPLC-Eluenten verdünnt.

### 2.3 Homogenisierung der Kontrollproben für konventionelle Analytik

### 2.3.1 Trocken vermahlene Proben (z.B. Getreide, wie Weizen)

Die restlichen circa 5 kg Lebensmittelbulkware werden, nachdem die in 2.1 beschriebene erfindungsgemäße Partikelprobe gewonnen wurde, aus dem Trommelmischer entnommen und direkt fein vermahlen (Korngröße < 1 mm). Diese Proben stellen die Kontrollen zu den durch die Vorrichtung gewonnen Partikelproben dar.

### 2.3.2 Nass vermahlene Proben (z.B. Rohkaffee, Nüsse o.ä., nicht jedoch Weizen, der sich durch das darin vorhandene Gluten nicht zum nass Vermahlen eignet, da sich ansonsten große Teigklumpen bilden würden)

Die restlichen ca. 5 kg Lebensmittelbulkware werden, nachdem die in 2.1 beschriebene erfindungsgemäße Partikelprobe gewonnen wurde aus dem Trommelmischer entnommen und gemäß dem amtlichen Untersuchungsverfahren nach § 64 LFBG L 00.00-111/1, mod. Untersuchung von Lebensmitteln - Probenvorbereitungsverfahren zur Bereitstellung der amtlichen Probe, Gegen- und Schiedsprobe für die Bestimmung des Mykotoxingehaltes in Lebensmitteln - Teil 1: - Verfahren zur Nasshomogenisierung homogenisiert. Diese Proben stellen die Kontrollen zu den durch die Vorrichtung gewonnenen Partikelproben dar.

### 2.4 Probenvorbereitung der Kontrollprobe zur Bestimmung der Mykotoxinkonzentration

10 g der nach 2.3.1 bzw. 2.3.2 homogenisierten Kontrollprobe werden mit 40 ml eine Acetonitril/Wasser-Gemisches (80:20; v:v) versetzt und 30 Minuten bei 175 U/min geschüttelt. Der Extrakt wird über einen Glasfaserfilter filtriert. 1 ml des filtrierten Extrakts werden mit 2 ml Wasser verdünnt, per Hand geschüttelt und mittels HPLC-MS/MS vermessen.

### 2.5 Messung der Extrakte der erfindungsgemäß entnommenen Partikelproben und der Kontrollproben

Die Messung der Extrakte erfolgt mittels HPLC gekoppelt mit einem Triple-Quadrupol-Massenspektrometer. Bei der stationären Phase handelt es sich um eine RP-18-Phase. Es wird ein binärer Gradient mit einem Eluent A und B eingesetzt. Die Flussrate beträgt 0,8 ml/min.

### Gradientenprogramm:

Eluent A: Wasser / Methanol (95+5; V+V), Ammoniumcarbonat: 1 mmol/L, Ameisensäure 0,0025%
Eluent B: Methanol

| Zeit (min) | Eluent A % | Eluent B % |
|---|---|---|
| 0,00 | 80 | 20 |
| 6,9 | 20 | 80 |
| 7,0 | 20 | 80 |
| 8,0 | 0 | 100 |
| 10,0 | 0 | 100 |
| 10,1 | 80 | 20 |
| 14,0 | 80 | 20 |

Die Detektion erfolgt sowohl im positiven (Ochratoxin A, Aflatoxin B1, T-2, HT-2) als auch im negativen (Deoxynivalenol, Zearalenon) Mode mittels des Triple-Quadrupol-Massenspektrometers als Sonderform der HPLC-MS.

### Beispiel 3: Analyse der Mykotoxine Deoxynivalenol (DON) und Zearalenon (ZON) in Weizen Schüttgut

Die Gewinnung der erfindungsgemäßen Partikelproben erfolgt gemäß Beispiel 1. Die Messung und die Bestimmung der Konzentration von DON bzw. ZON in den erfindungsgemäßen Partikelproben sowie in den konventionell gewonnenen Kontrollproben erfolgt gemäß Beispiel 2. Die Ergebnisse der Bestimmung der Konzentration von DON bzw. ZON als Mykotoxine in den erfindungsgemäßen Partikelproben und in den Kontrollproben sind in der folgenden Tabelle 1 dargestellt:

**Tabelle 1:**

| | **Deoxynivalenol (DON)** | | **Zearalenon (ZON)** | |
|---|---|---|---|---|
| **Proben-Nr.** | **Partikelprobe** | **Kontrollprobe** | **Partikel probe** | **Kontrollprobe** |
| | **µg/kg** | | **µg/kg** | |
| **Weizen** | | | | |
| **673517** | 826,7 | 100 | 326,7 | 24 |
| **673518** | 1500 | 290 | 1040 | 23 |
| **673519** | 580 | 100 | 414 | 13 |
| **673521** | 2100 | 330 | 732 | 20 |
| **673522** | 1500 | 200 | 412 | 10 |
| **674444** | 950 | 120 | 144,3 | 10 |
| **676177** | 770 | 140 | 64,9 | 14 |
| **675915** | 460 | 62 | 17,4 | 0,3 |
| **676398** | 1100 | 190 | 1180 | 13 |
| **677547** | 4700 | 650 | 4220 | 110 |
| **678064** | 17000 | 1400 | 2710 | 94 |
| **682371** | 470 | 54 | 79 | 1 |
| **687390** | 260 | 13 | 19,8 | 1 |
| **687480** | 4500 | 410 | 2030 | 48 |
| **689724** | 310 | 15 | 16,9 | 0,1 |
| **718344** | 842 | 200 | 270 | 13 |

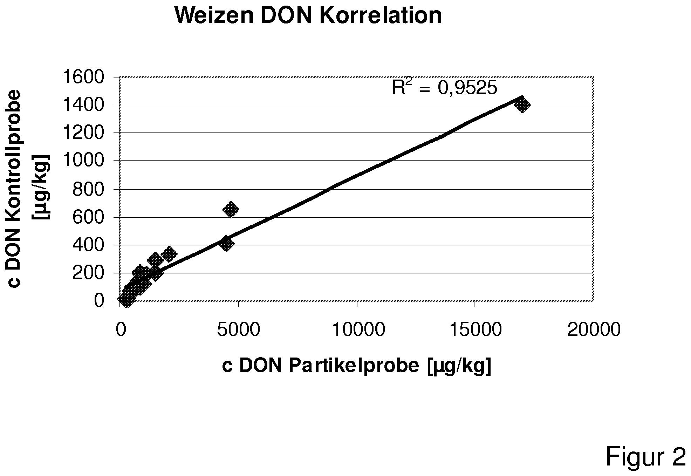

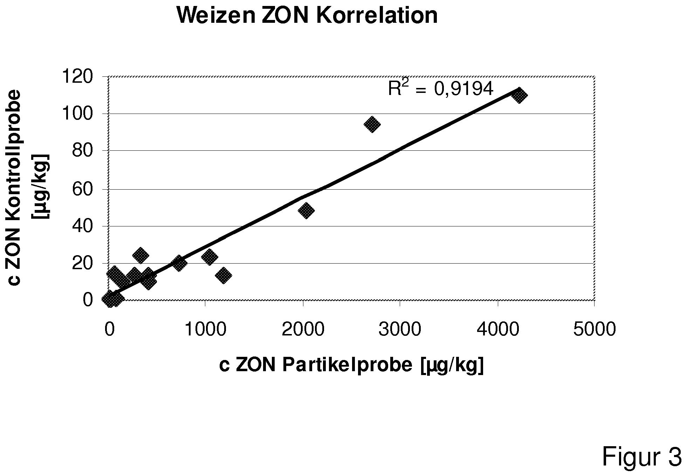

Die Ergebnisse der Konzentrationsbestimmung sind in Figur 2 und 3 dargestellt. Überraschenderweise konnten die Erfinder feststellen, dass der Nachweis der Mykotoxine in den durch die Vorrichtung gewonnenen Partikelproben in einem hohen Maße mit den Ergebnissen der aus Gesamtmaterial gewonnenen Kontrollproben korreliert. Aus Figur 2 geht eine Korrelation von R² = 0,9525 der Konzentration von DON in den erfindungsgemäßen Partikelproben und den Kontrollproben hervor. Da eine perfekte Korrelation bei einem Wert von R² = 1 anzunehmen ist, stellt der Wert von R² = 0,9525 eine sehr hohe Korrelation dar. Insofern stellt die erfindungsgemäße Partikelprobe eine sehr repräsentative Probe dar, die in einem verlässlichen Ausmaß die Konzentration von DON als Mykotoxin in der Gesamtprobe wiedergibt. Aus Figur 3 geht hervor, dass die Korrelation der Konzentration von ZON in Weizen der erfindungsgemäßen Partikelproben mit den Kontrollproben R² = 0,9194 beträgt. Somit ist die erfindungsgemäß erhaltene Probe auch in einem hohen Ausmaß aussagekräftig im Hinblick auf die gemessene Konzentration des Mykotoxins ZON in der Kontrollprobe.

### Beispiel 4: Analyse der Mykotoxine Deoxynivalenol (DON) und Zearalenon (ZON) in Mais Schüttgut

Die Gewinnung der erfindungsgemäßen Partikelproben erfolgt gemäß Beispiel 1. Die Messung und die Bestimmung der Konzentration von DON bzw. ZON in den erfindungsgemäßen Partikelproben sowie in den konventionell gewonnenen Kontrollproben erfolgt gemäß Beispiel 2. Die Ergebnisse der Messung der Konzentrationen von DON bzw. ZON in den erfindungsgemäßen Partikelproben und in den konventionell gewonnenen Kontrollproben sind in der folgenden Tabelle 2 dargestellt:

**Tabelle 2:**

| Parameter | **DON** | | **ZON** | |
|---|---|---|---|---|
| **Probe-Nr.** | **Partikel probe** | **Kontrollprobe** | **Partikelprobe** | **Kontrollprobe** |
| | **µg/kg** | | **µg/kg** | |
| **Mais** | | | | |
| **684007** | 110 | 10 | 9,73 | 0,4 |
| **688723** | 440 | 14 | 78,2 | 2,5 |
| **688726** | 410 | 21 | 44,4 | 0,4 |
| **Mais_Lab** | 2000 | 260 | 272 | 27 |
| **689723** | 5100 | 450 | 730 | 32 |
| **691361** | 840 | 61 | 60,9 | 1,2 |
| **692053** | 150 | 25 | 9,1 | 2,9 |
| **692054** | 210 | 35 | 5,4 | 3,1 |
| **692055** | 605 | 42 | 22,1 | 3,1 |

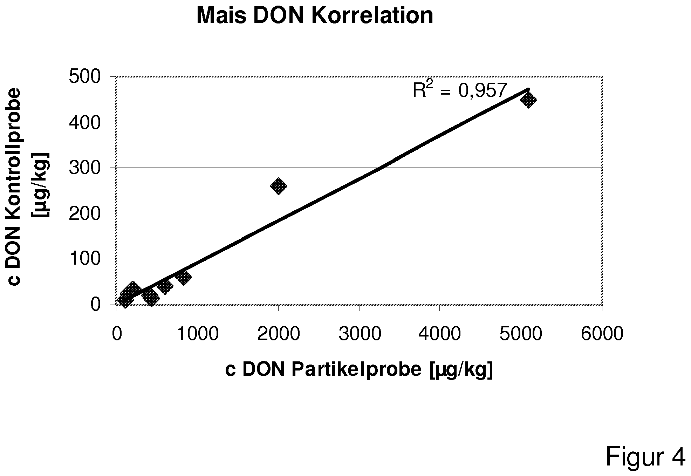

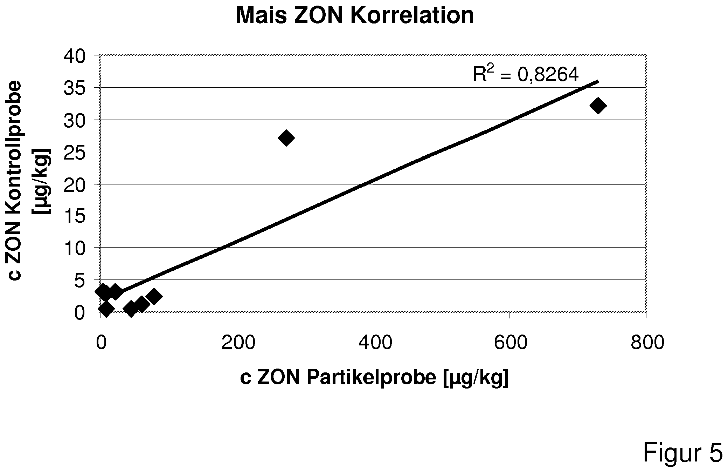

Auch für die Konzentrationen von DON bzw. ZON in Mais als Lebensmittelschüttware konnten die Erfinder überraschenderweise zeigen, dass zwischen den erfindungsgemäß gewonnenen Partikelproben und den aus der direkt vermahlenen Kontrollprobe aus dem Mais-Schüttgut eine hohe Korrelation vorliegt. Figur 4 zeigt eine Korrelation von R² = 0,957 für die Konzentration von DON. Somit ist die durch die Vorrichtung gewonnene Probe in einem hohen Maße aussagekräftig und damit repräsentativ, um auf so eine Belastung von Mais mit dem Mykotoxin DON verlässlich und auf einfache Weise festzustellen. Gleiches trifft auch auf die Messung der Konzentration mit ZON zu. Figur 5 zeigt eine hohe Korrelation von R² = 0,8264. Somit ist auch für die Bestimmung dieses weiteren Mykotoxins ZON belegt, dass durch die Vorrichtung eine in ihrer Aussagekraft verlässliche und repräsentative Probe gewonnen werden kann. Im Vergleich zu den zeitaufwendigen und technisch problematischen Proben, die durch die komplette Homogenisierung der Schüttware gewonnen werden müssen, stellt somit die erfindungsgemäß gewonnene Probe aus den Partikeln der Schüttware eine sichere und technisch leichter umzusetzende Alternative dar, die zudem eine deutliche Zeit- und Kostenersparnis bewirkt und gleichzeitig vergleichbare Resultate hinsichtlich der Mykotoxinkonzentration aufweist.

### Beispiel 5: Analyse der Mykotoxine Ochratoxin A (OTA) in Kaffee Schüttgut

Die Gewinnung der erfindungsgemäßen Partikelproben erfolgt gemäß Beispiel 1. Die Messung und die Bestimmung der Konzentration von Ochratoxin A (OTA) in den erfindungsgemäßen Partikelproben sowie in den konventionell gewonnenen Kontrollproben erfolgt gemäß Beispiel 2. Die Ergebnisse der Messung der Konzentrationen von Ochratoxin A (OTA) in den erfindungsgemäßen Partikelproben und in den konventionell gewonnenen Kontrollproben sind in der folgenden Tabelle 3 dargestellt:

**Tabelle 3:**

| | **Ochratoxin A (OTA)** | **Ochratoxin A (OTA)** |
|---|---|---|
| **Proben-Nr.** | **Partikel probe** | **Kontrollprobe** |
| | **[µg/kg]** | **[µg/kg]** |
| **Kaffee** | | |
| **683699** | 40,1 | 1,2 |
| **683700** | 50,5 | 2,0 |
| **684131** | 20,4 | 1,7 |
| **684132** | 25,7 | 0,5 |
| **684970** | 24,4 | 0,8 |
| **685794** | 22,3 | 0,7 |
| **685795** | 15,1 | 0,2 |
| **685796** | 10,8 | 0,6 |
| **686195** | 16,1 | 1,0 |
| **kaffee_Lab** | 26,1 | 0,0 |
| **686652** | 63,5 | 2,0 |
| **686653** | 20,6 | 0,3 |
| **687580** | 65,5 | 4,0 |
| **687951** | 11,6 | 1,0 |
| **687952** | 47,6 | 1,9 |
| **687953** | 39,3 | 1,8 |
| **687954** | 51,5 | 1,8 |
| **688832** | 65,7 | 5,3 |
| **689831** | 75,7 | 3,2 |
| **688425** | 20,4 | 1,1 |
| **690546** | 50,9 | 2,3 |
| **690547** | 37,4 | 0,0 |
| **690969** | 54,1 | 1,1 |
| **690970** | 36,3 | 1,9 |
| **694987** | 17,8 | 0,0 |
| **694988** | 32,0 | 0,0 |
| **694525** | 12,3 | 0,0 |
| **701399** | 23,37 | 0,70 |
| **706186** | 28,39 | 0,00 |
| **706187** | 58,88 | 2,40 |
| **706495** | 36,53 | 0,60 |
| **706496** | 43,26 | 0,70 |
| **707928** | 17,15 | 0,00 |
| **708735** | 16,56 | 2,00 |
| **709169** | 30,87 | 1,00 |
| **709710** | 19,10 | 1,30 |
| **710087** | 25,83 | 0,50 |
| **711828** | 35,83 | 0,80 |
| **712250** | 22,37 | 0,50 |
| **712249** | 35,82 | 0,30 |
| **712639** | 38,35 | 0,70 |

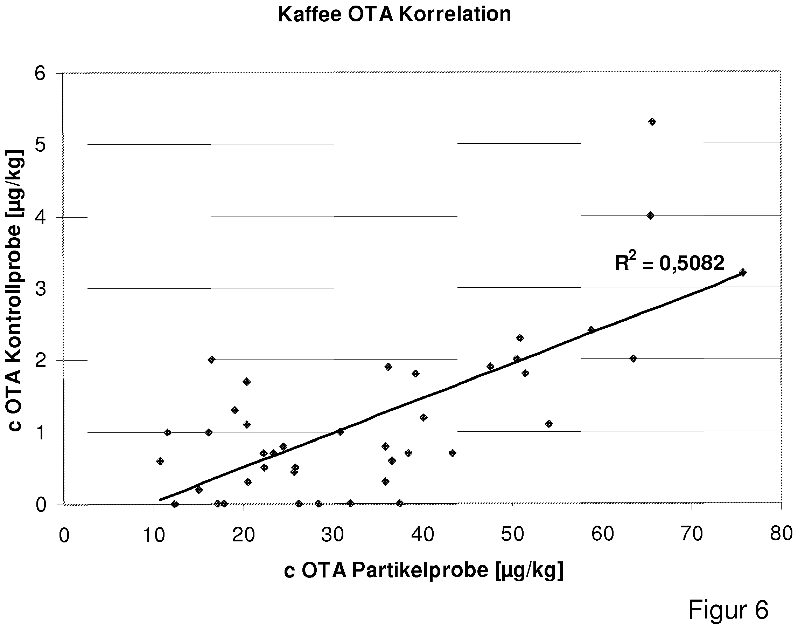

Die Messwerte aus Tabelle 3 zeigen eine Korrelation zwischen der Belastung mit Ochratoxin A (OTA) in den erfindungsgemäßen Partikelproben mit den Kontrollproben. Diese Korrelation ist in Figur 6 dargestellt. Die Korrelation beträgt R² = 0,5082. Dieser Wert ist als relativ gering einzustufen. Ursache hierfür ist eine geringere Gesamtbelastung des untersuchten Kaffee-Schüttguts. Anhand der Daten kann jedoch der Rückschluss gezogen werden, dass eine OTA Belastung der erfindungsgemäßen Partikelprobe von < 50 µg/kg eine Gesamtbelastung der Gesamtprobe mit OTA von < 5 µg/kg bedeutet.

## Patentansprüche

1. Verfahren zum Nachweis mindestens einer Mykotoxinkontamination in Schüttgut umfassend die Schritte:
(a) Entnehmen einer Probe von Partikeln mit einer Partikelgröße von 0,1 mm bis 1,0 mm aus Schüttgut;
(b) Analysieren der entnommenen Probe;
(c) Nachweis mindestens einer Mykotoxinkontamination durch Bestimmen der Konzentration der Mykotoxinkontamination, und
(d) Rückschluss auf die tatsächliche Mykotoxinkontamination des Schüttguts durch Korrelieren der in Schritt (c) bestimmten Konzentration in der Probe mit derjenigen einer Gesamtprobe des Schüttguts.

2. Verfahren nach Anspruch 1, wobei die Partikelgröße der entnommenen Probe eine Größe von 0,1 mm bis 0,7 mm, weiter bevorzugt von 0,2 mm bis 0,5 mm aufweist.

3. Verfahren nach einem der Ansprüche 1 oder 2, wobei die Analyse durch eine Analysevorrichtung zur Durchführung von chromatographischen, spektroskopischen oder immunologischen Verfahren erfolgt.

4. Verfahren nach Anspruch 1 bis 3, wobei die Analyse mittels High Performance Liquid Chromatography (HPLC), Ultra-High-Performance Chromatography (UPLC), Fourier-Transformations-IR-Spektroskopie (FTIR), Kernspinresonanzspektroskopie (nuclear magnetic resonance, NMR), Direct Analysis in Real Time (DART) oder Enzyme-linked immunosorbent assay (ELISA) durchgeführt wird.

5. Verfahren nach einem der Ansprüche 1 bis 4, wobei die Mykotoxine ausgewählt sind aus der Gruppe bestehend aus Ochratoxinen, Aflatoxinen, Mutterkornalkaloiden, Fusarium-Toxinen, wie insbesondere bevorzugt Deoxynivalenol und Zearalenon, Alternaria-Toxinen und Citrinin.

6. Verfahren nach einem der Ansprüche 1 bis 5, wobei die Bestimmung der Mykotoxinkontamination im Schüttgut mit Hilfe einer Korrelation mit Kontrolldaten aus der in der Partikelprobe bestimmten Konzentration erfolgt.

## Claims

1. Method for the detection of at least one mycotoxin contamination in bulk ware, the method comprising the steps of:
(a) taking a sample of particles with a particle size of 0.1 mm to 1.0 mm from bulk ware;
(b) analysing of the taken sample;
(c) detecting at least one mycotoxin contamination by determining of the concentration of the mycotoxin contamination, and
(d) inferring the actual mycotoxin contamination of the bulk ware by correlating the concentration in the sample as determined in step (c) with the concentration of the overall sample of the bulk ware.

2. Method according to claim 1, wherein the particle size of the extracted sample has a size of 0.1 mm to 0.7 mm, further preferred of 0.2 mm to 0.5 mm.

3. Method according to any one of claims 1 or 2, wherein the analysis is made by an analysis device for the conduction of chromatographic, spectroscopic, or immunological procedures.

4. Method according to claims 1 to 3, wherein the analysis is conducted by High Performance Liquid Chromatography (HPLC), Ultra-High-Performance Chromatography (UPLC), Fourier-Transformations-IR-Spectroscopy (FTIR), Nuclear magnetic resonance (NMR), Direct Analysis in Real Time (DART) or enzyme-linked immunosorbent assay (ELISA).

5. Method according to any one of claims 1 to 4, wherein the mycotoxins are selected from the group consisting of ochratoxins, Aflatoxins, ergot alkaloids, fusarium toxins, in particular preferred the deoxynivalenol and zearalenone, alternia toxins and citrinin.

6. Method according to any one of claims 1 to 5, wherein determining of the mycotoxin contamination within the bulk ware is made by means of a correlation with control data from the determined concentration in the sample of particles.

## Revendications

1. Procédé de détection d'au moins une contamination par mycotoxine dans de la marchandise en vrac, comprenant les étapes de :
(a) prélèvement d'un échantillon de particules d'une taille de particules de 0,1 mm à 1,0 mm dans de la marchandise en vrac ;
(b) analyse de l'échantillon prélevé ;
(c) détection d'au moins une contamination par mycotoxine en déterminant la concentration de la contamination par mycotoxine, et
(d) conclusion quant à la contamination par mycotoxine réelle de la marchandise en vrac par corrélation de la concentration dans l'échantillon déterminée à l'étape (c) avec celle d'un échantillon global de la marchandise en vrac.

2. Procédé selon la revendication 1, dans lequel la taille de particules de l'échantillon prélevé présente une taille de 0,1 mm à 0,7 mm, plus préférentiellement de 0,2 mm à 0,5 mm.

3. Procédé selon l'une des revendications 1 ou 2, dans lequel l'analyse est réalisée par un dispositif d'analyse permettant de réaliser des procédés chromatographiques, spectrométriques ou immunologiques.

4. Procédé selon la revendication 1 à 3, dans lequel l'analyse est réalisée par chromatographie liquide haute performance (HPLC), chromatographie ultra-haute-performance (UPLC), spectrométrie IR à transformée de Fourier (FTIR), spectrométrie à résonance magnétique nucléaire (NMR), analyse directe en temps réel (DART) ou méthode immuno-enzymatique (ELISA).

5. Procédé selon l'une des revendications 1 à 4, dans lequel les mycotoxines sont choisies dans le groupe constitué des ochratoxines, des aflatoxines, des alcaloïdes de l'ergot de seigle, des toxines de fusarium, comme notamment de manière préférée le désoxynivalénol et la zéaralénone, les toxines d'Alternaria et la citrinine.

6. Procédé selon l'une des revendications 1 à 5, dans lequel la détermination de la contamination par mycotoxine dans la marchandise en vrac s'effectue à l'aide d'une corrélation avec des données témoins provenant de la concentration déterminée dans l'échantillon de particules.
